(19) Europäisches Patentamt
European Patent Office
Office européen des brevets

(11) Publication number: 0 227 331 B1

# (12) EUROPEAN PATENT SPECIFICATION

(45) Date of publication of patent specification: 01.04.92

(51) Int. Cl.5: C07C 49/825, C07C 45/54, B01J 29/18, B01J 29/28

(21) Application number: 86309339.9

(22) Date of filing: 01.12.86

## (54) Process for producing 2-hydroxyphenyl lower alkyl ketones.

(30) Priority: 02.12.85 US 803194
02.12.85 US 803195

(43) Date of publication of application:
01.07.87 Bulletin 87/27

(45) Publication of the grant of the patent:
01.04.92 Bulletin 92/14

(84) Designated Contracting States:
AT BE CH DE ES FR GB GR IT LI LU NL SE

(56) References cited:
US-A- 2 833 825
US-A- 3 354 221
US-A- 3 702 886
US-A- 4 061 724

(73) Proprietor: CELANESE CORPORATION
1211 Avenue of the Americas
New York New York 10036(US)

(72) Inventor: Gupta, Balaram B.G.
401 Route 22, Appartment 36F
North Plainfield New Jersey(JP)
Inventor: Nicolau, Ioan
4522 Acushnet
Corpus Christi Texas(US)
Inventor: Aguilo, Adolfo
4604 Oxford
Corpus Christi Texas(US)

(74) Representative: De Minvielle-Devaux, Ian
Benedict Peter et al
CARPMAELS & RANSFORD 43, Bloomsbury
Square
London WC1A 2RA(GB)

EP 0 227 331 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to a process for producing 2-hydroxyphenyl lower alkyl ketones such as 2-hydroxyacetophenone.

Compounds such as 2-hydroxyphenyl lower alkyl ketones, e.g. 2-hydroxyacetophenone (2-HAP) are possible intermediates for a variety of products having different end uses. Thus, 2-HAP may be converted into catechol (1,2-dihydroxybenzene) by first reaoting the 2-HAP to form the monoacetate ester of catechol using a "Baeyer-Villiger" oxidation as disclosed, for example in European Patent Application 85307489.6, filed 17 October 1985 and published as EP-A-178,929, and the references cited therein, and then converting the monoacetate ester to catechol by hydrolysis, e.g. as disclosed in the previously cited EP-A-178,929, or by transesterification as disclosed in European Patent Application 86300041.0, filed 6 January 1986 and published as EP-A-190,815, and the references cited therein. Alternatively, the 2-HAP can be converted into guaiacol by first methylating it to form 2-methoxyacetophenone, and then obtaining the acetate ester of the monomethyl ether of catechol by a Baeyer-Villiger oxidation and the guaiacol by hydrolysis or transesterification as previously described for catechol. Aspirin may be made from 2-HAP by first acetylating it with acetic anhydride to yield 2-acetoxyacetophenone and oxidizing the latter compound with a transition metal catalyst to form aspirin; these reactions are disclosed in European Patent Application 85305222.3, filed 23 July 1985 and published as EP-A-170,483, and the references cited therein. The two following published references teach the reaction of phenol and acetic acid to form 4-hydroxyacetophenone (4-HAP), as described:

Dann and Mylius in a dissertation included as part of a series of Reports from the Institute for Applied Chemistry of the University of Erlangen, received for publication on January 7, 1954 and published in Annalen der Chemie 587 Band, pages 1 to 15, disclose the reaction of phenol and glacial acetic acid in the presence of hydrogen fluoride to produce 4-hydroxyacetophenone (4-HAP) in a yield of 61.6%. This reaction may be conventionally characterized as a Friedel-Crafts acetylation of phenol with acetic acid as the acetylating agent.

Simons et al, Journal of the American Chemical Society, 61, 1795 and 1796 (1939) teach the acylation of aromatic compounds using hydrogen fluoride as a condensing agent and in Table 1 on page 1796 show the acetylation of phenol with acetic acid to produce p-hydroxyacetophenone (4-HAP) in 40% yield.

None of the foregoing disclosures, however, teach any method of reacting phenol with acetic acid to obtain a product comprising a preponderance of 2-HAP, or of carrying out such reaction in the presence of an H-ZSM-5 zeolite or silicalite catalyst to obtain a product comprising 2-HAP.

The prior art also discloses the Fries rearrangement of phenyl acetate to form hydroxyacetophenones. For example, US-A-3,354,221 (Landis et al.) discloses a method of converting a phenolic ester, such as phenyl acetate, into a ketonic phenol, such as o- or p-hydroxyacetophenone, in the presence of a solid porous crystalline aluminosilicate zeolite catalyst having pores of a size sufficient to permit ingress of the phenolic ester and egress of the ketonic phenol. There is, however, no disclosure therein of the use of an H-ZSM-5 zeolite or silicalite catalyst.

The previously cited Dann and Mylius article shows the rearrangement of phenyl acetate in hydrogen fluoride to 4-hydroxyacetophenone, with a maximum yield of 81% after 24 hours of reaction time, and reports a yield of 92% stated to be obtained by K. Weichert as reported in Angewandte Chemie 56, 338 (1943). Although Dann and Mylius suggest that the difference in yields may be at least partly due to the previous ignoring by Weichert of the accompanying 2-hydroxyacetophenone, the latter is nevertheless produced in only minor amounts as compared with 4-hydroxyacetophenone.

Davenport et al, U.S. Patent No. 4,524,217, disclose a process for the production of N-acetyl-para-aminophenol (acetoaminophen) including the Fries rearrangement of phenyl acetate with a hydrogen fluoride catalyst to product a preponderance of 4-hydroxyacetophenone.

Thus, none of these references discloses a method for carrying out a Fries rearrangement of phenyl acetate in the presence of a silicalite catalyst to produce 2-hydroxyacetophenone.

Various zeolites and zeolite-type materials are known in the art for the catalysis of chemical reactions. For example, U.S. Patent No. 3,702,886, of Argauer et al., discloses a class of synthetic zeolites, characterized as "Zeolite ZSM-5", which are effective for the catalysis of various hydrocarbon conversion processes.

## SUMMARY OF THE INVENTION

In accordance with one aspect of this invention, phenol is reacted with a lower alkanoic acid, e.g. acetic acid, in the presence of an at least partially protonated ZSM-5 zeolite-type catalyst, characterized as an "H-ZSM-5" catalyst, or in the presence of a silicalite catalyst containing alumina ($Al_2O_3$) within a defined range as hereinafter defined, said silicalite catalyst having been calcined from the as synthesized form at least once, to produce a product comprising a 2-hydroxyphenyl lower alkyl ketone, e.g. 2-hydroxyacetophenone (2-HAP). The exact amount of the 2-hydroxyphenyl ketone in the product may vary depending on conditions with most of the remainder being the corresponding 4-hydroxyphenyl ketone, e.g. 4-hydroxyacetophenone (4-HAP).

In accordance with another aspect of the invention, an ester of phenol and a lower alkanoic acid, e.g. phenyl acetate, is reacted in contact with a silicalite catalyst to produce a product comprising a 2-hydroxyphenyl lower alkyl ketone, e.g. 2-HAP.

The reaction of phenol and alkanoic acid proceeds in accordance with the following equation:

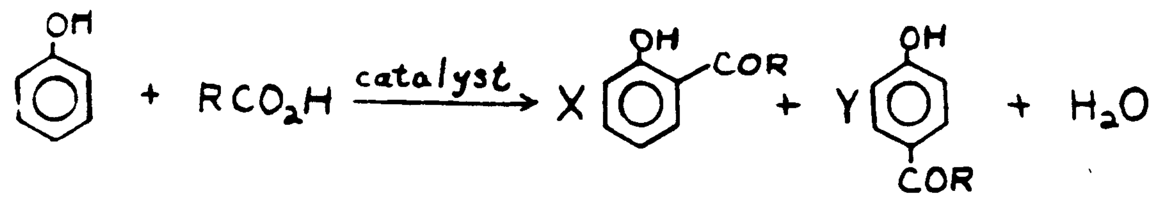


where R is lower alkyl, and X and Y add up to 1 and are the mole fractions, or expressed as percentages, the selectivities of the 2-hyroxyphenyl and 4-hydroxyphenyl ketones respectively based on the total amount of hydroxyphenol ketones produced in the reaction.

If the lower alkanoic acid is acetic acid, i.e., R is methyl, the reaction proceeds as in the following equation:

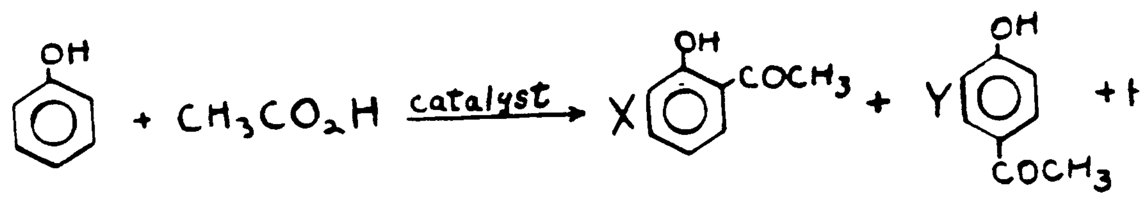


The reaction utilizing a silicalite catalyst of an ester of phenol with a lower alkanoic acid may be assumed to be a Fries rearrangement which proceeds in accordance with the following equation:

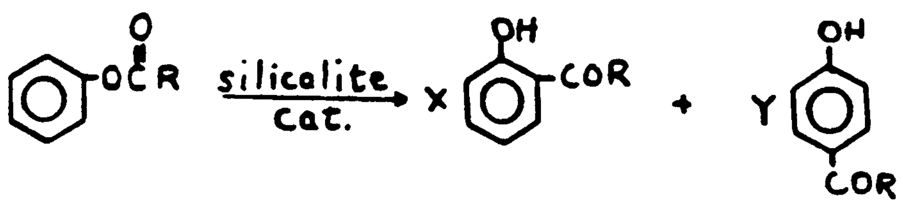


where R, X and Y are the definitions given previously.

If the ester reacted is phenyl acetate, the reaction proceeds as follows:

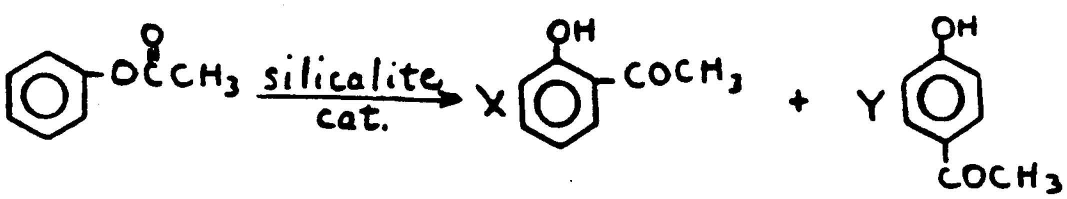


Preferably, R in the foregoing equations contains 1 to 3 carbon atoms so that the lower alkanoic acid in free form or as the acid forming the ester with phenol is acetic, propionic, n-butyric or isobutyric acid. The preferred acid is acetic acid which yields 2-HAP as the preferred product.

If an ester of phenol and a lower alkanoic acid is used as the starting material with a silicalite as

catalyst, the reaction may be carried out in the presence of a free lower alkanoic acid, e.g. acetic acid as an additive for the reaction. The acid may be the same or different from the acid used to prepare the phenolic ester starting material.

The H-ZSM-5 zeolites utilized as catalysts in the process of this invention are prepared by replacing with hydrogen ions most of the cations of a ZSM-5 zeolite, the composition, characteristics and preparation of which are set out in the previously cited U.S. Patent No. 3,702,886 of Argauer et al.,. These ZSM-5 zeolites have the following formula.

$0.9 \pm 0.2M_{2/n}O:W_2O_3:5\text{-}100\ YO_2:zH_2O$

wherein M is a cation, n is the valence of said cation, W is selected from the group consisting of aluminum and gallium, Y is selected from the group consisting of silicon and germanium, and z is from 0 to 40. In a preferred synthesized form, the zeolite has a formula, in terms of mole ratios of oxides, as follows:

$0.9 \pm 0.2M_{2/n}O:Al_2O_3:5\text{-}100\ SiO_2:zH_2O$

and M is selected from the group consisting of a mixture of alkali metal cations, especially sodium, and tetraalkyl-ammonium cations, the alkyl groups of which preferably contain 2-5 carbon atoms. In a particularly preferred class of catalysts for purposes of the present invention, the molar ration of $SiO_2$ to $Al_2O_3$ in the latter formula is within the ratio of about 10 to 60.

The ZSM-5 zeolites in most cases have a distinguishing crystalline structure yielding an X-ray diffraction pattern determined as described in U.S. Patent No. 3,702,886, which significant lines as indicated in Table I, wherein "s" = strong, "w" = weak and "v.s." = very strong.

**TABLE I**

| Interplanar spacing d(A): | Relative intensity |
|---|---|
| 11.1 ± 0.2 | s. |
| 10.0 ± 0.3 | s. |
| 7.4 ± 0.15 | w. |
| 7.1 ± 0.15 | w. |
| 6.3 ± 0.1 | w. |
| 6.04 ± <br> 5.97 ± 0.1 | w. |
| 5.56 ± 0.1 | w. |
| 5.01 ± 0.1 | w. |
| 4.60 ± 0.06 | w. |
| 4.25 ± 0.06 | w. |
| 3.85 ± 0.07 | v.s. |
| 3.71 ± 0.05 | s. |
| 3.04 ± 0.03 | w. |
| 2.99 ± 0.02 | w. |
| 2.94 ± 0.02 | w. |

The "H-ZSM-5" catalyst utilized in the process of the present invention is prepared from a "ZSM-5" zeolite by replacing most, and generally at least about 80%, of the cations of the latter zeolite with hydrogen ions using techniques well-known in the art.

The silicalites utilized as catalysts in the process of this invention are crystalline silica polymorphs, many of which are similar to those described, for example, in U.S. Patent No. 4,061,724, issued to Grose et al on December 7, 1977.

The X-ray powder diffraction pattern of many of the silicalites utilized in the present invention (600° C. calcination in air for one hour) has as its six strongest lines (i.e. interplanar spacings) those set forth in Table II below, wherein "S" = strong and "VS" = very strong.

TABLE II

| d-A | Relative Intensity |
|---|---|
| 11.1 ± 0.2 | VS |
| 10.0 ± 0.2 | VS |
| 3.85 ± 0.07 | VS |
| 3.82 ± 0.07 | S |
| 3.76 ± 0.05 | S |
| 3.72 ± 0.05 | S |

The silicalites utilized as catalysts in the present invention have in the as-synthesized form a specific gravity at 25° C. of 1.99 ± 0.08 cc as measured by water displacement. In the calcined (600° C. in air for 1 hour) form silicalite has a specific gravity of 1.70 ± 0.08 g/cc. With respect to the mean refractive index of silicalite crystals, values obtained by measurement of the as-synthesized form and the calcined form (600° C. in air for 1 hour) are, respectively, 1.48 ± 0.01 and 1.39 ± 0.01.

Crystals of silicalite in both the as-synthesized and calcined form are orthorhombic and have the following unit cell parameters: a = 20.05 A, b = 20.0 A, c = 13.4 A, with an accuracy of ± 0.1 A on each of the above values. The pore diameter of silicalite is above 5 to 6 Angstrom units and its pore volume is 0.18 ± 0.02 cc./gram as determined by absorption. (1 Angstrom, A = 0.1 nm.)

The pores of the silicalite particles have a pattern providing for easy access to vapors and liquids intended to be treated. For example the pores may be in the form of zig-zag channels cross-linked by straight channels.

The preparation of silicalite may be accomplished, for example by the hydrothermal crystallization of a reaction mixture comprising water, a source of silica and an alkylonium compound at a pH of 10 to 14 to form a hydrous crystalline precursor, and subsequently calcining that precursor to decompose alkylonium moieties present therein.

The alkylonium cation is suitably supplied to the reaction system by a compound preferably soluble in the reaction micture and which contains a quaternary cation generally expressed by the formula:

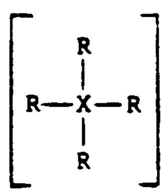

wherein R is an alkyl radical containing from 2 to 6 carbon atoms and X represents either phosphorus or nitrogen. Preferably R is ethyl, propyl or n-butyl, especially propyl, and X is nitrogen. Illustrative compounds include tetraethylammonium hydroxide, tetrapropylammonium hydroxide, tetrabutylammonium hydroxide, tetrabutylphosphonium hydroxide and the salts corresponding to the aforesaid hydroxides, particularly the chloride, iodide and bromide salts, for example, tetrapropylammonium bromide. The quaternary compounds can be supplied to the reaction mixture per se or can be generated in situ, such as by the reaction of tertiary amines with alkyl halides or sulfates.

When the quaternary cation is provided to the system in the form of the hydroxide in sufficient quantity to establish a basicity equivalent to the pH of 10 to 14, the reaction mixture need contain only water and a reactive form of silica as additional ingredients. In those cases in which the pH is required to be increased to above 10, ammonium hydroxide or alkali metal hydroxides can be suitably employed for that purpose, particularly the hydroxides of lithium, sodium or potassium. It has been found that not more than 6.5 moles of alkali metal oxide per mole-ion of quaternary cation is required for this purpose even if none of the quaternary cation is provided in the form of its hydroxide.

The source of silica in the reaction mixture can be wholly or in part alkali metal silicate but should not be employed in amounts greater than that which would change the molar ratio of alkali metal to quaternary cations set forth above. Other silica sources include reactive amorphous silica such as fumed silica, silica sols and silica gel.

The silicalites contemplated for use in this invention generally contain about 700 to 14,000 ppm of alumina corresponding to a $SiO_2$ to $Al_2O_3$ ratio of about 120 to 1450, preferably about 5000 to 7000 ppm of

alumina corresponding to a $SiO_2$ to $Al_2O_3$ ratio of about 240 to 340. The amount of alumina in the silicalite will generally depend to a large extent of the source of silica. For example, commercially available silica sols can typically contain from 500 to 700 ppm $Al_2O_3$, whereas fumed silicas can contain from 80 to 2000 ppm of $Al_2O_3$. Silicalites containing still larger amounts of alumina may be obtained by using other sources of silica with higher contents of alumina as is well-known in the art.

The quantity of silica in the reaction system should be from about 13 to 50 moles $SiO_2$ per mole-ion of the quaternary cation. Water should be present in an amount of from 150 to 700 moles per mole-ion of the quaternary cation.

Accordingly, in preparing the crystalline silicalite precursor, there is formed a reaction mixture having a pH of at least 10 which in terms of moles of oxides contains from 150 to 700 moles $H_2O$, from 13 to 50 moles non-crystalline $SiO_2$ and from 0 to 6.5 moles $M_2O$, wherein M is an alkali metal, for each mole of $Q_2O$ present, wherein Q is a quaternary cation having the formula $R_4X+$ in which each R represents hydrogen or an alkyl group containing from 2 to 6 carbon atoms and X is phosphorus or nitrogen.

The order in which the reagents are admixed is not a critical factor. The reaction mixture is maintained at a temperature of from about 100 to 250° C. under autogeneous pressure until crystals of the silicalite precursor are formed, ordinarily from about 50 to 150 hours. The crystalline product is recovered by any convenient means such as filtration. Advantageously the product is washed with water and can be dried in air at about 100°C.

When alkali metal hydroxide has been employed in the reaction mixture, alkali metal moieties appear as impurities in the cyrstalline product. Although the form in which these impurities exist in the crystalline mass has not yet been determined, they are not present as cations which undergo reversible exchange. The quaternary cation moiety is quite readily thermally decomposed and removed by calcination in an oxidizing atmosphere (air) or inert atmosphere at temperatures of from about 480°C. to 1000° C. for a period sufficient to obtain the desired silicalite, usually, about 1 to 6 hours. The residual alkali metal in the product can be removed or reduced by washing with alkali metal halide solution or in aqueous acid solution of sufficient strength such as hydrochloric acid. The crystal structure is not otherwise affected by contact with strong mineral acids even at elevated temperature due to the lack of acid-soluble constituents in its crystal structure.

The reaction may be carried out in the vapor or liquid phase under a wide variety of conditions. Reaction temperatures may be employed, for example in the range of about 160 to 350° C., preferably about 200 to 300° C. when an H-ZSM-5 zeolite is used as catalyst, and about 200 to 350° C., preferably about 250 to 320° C., when a silicalite is used as catalyst. The pressure is generally uncritical to the reaction and subatmospheric, atmospheric or superatmospheric pressures may be employed. In most cases, however, the pressure of the reaction will be in the range of about 1 to 30 atmospheres absolute (101 to 3,040 kPa) when an H-ZSM-5 zeolite is used, and about 1 to 25 atmospheres absolute (101 to 2,533 kPa) when a silicalite is used as catalyst. Generally, it is most advantageous to carry out the reaction in the vapor phase, in which case when a silicalite is used as catalyst, the temperature may be, for example, in the range of about 250 to 320° C., preferably about 280 to 300° C. at a pressure, for example, of about 1 to 3 atmospheres (101 to 304 kPa), more preferably about 1 to 2 atmospheres (101 to 203 kPa), and most preferably about 1.5 to 1.7 atmospheres (152 to 172 kPa).

Contact or residence time can also vary widely, depending upon such variables as the lower alkanoic acid, catalyst, reactor, temperature and pressure. Typical contact times range from a fraction of a second to more than several hours when a catalyst system other than an fixed bed is used, with preferred contact times, at least for vapor phase reactions, between about 0.5 and 100 seconds when an H-ZSM-5 zeolite is used and about 3 to 25, preferably about 5 to 15 seconds when a silicalite is used as catalyst.

It has been found that the presence of water in the reactant feed stream when an H-ZSM-5 zeolite is used as catalyst, affects the yield of 2-hydroxyphenyl ketone produced. Water is also a principal by-product of the reaction. The molar ratio of water to phenol can range as high as 2 and above. However, an improvement in conversions of phenol and acid can be obtained with concomitant high selectivity to 2-hydroxyphenyl ketones with molar ratios of water to phenol as low as 0.5. Thus, the amount of water utilized can range from about 0.5 mole up to about 2 moles of water per mole of phenol and, preferably, ranges from about 1 to 2 moles of water per mole of phenol feed.

Although the reaction between phenol and lower alkanoic acid consumes one mole of phenol per mole of acid to produce a mole of hydroxypheny lower alkyl ketones, the actual molar ratio of phenol to alkanoic acid in the feed stream may be varied between wide limits, e.g. from about 100:1 to 1:100. It is preferred however that such ratio be in the range of about 1:20 to 1:1.

A lower alkanoic acid when employed as an additive or solvent for the reaction may be added, for example, in an amount of about 1 to 5 moles, preferably about 1 to 2 moles per mole of phenol or phenol

ester in the feed.

If the feed compounds are in the vapor state at the reaction temperature, then they can be fed in undiluted state or diluted with a relatively inert carrier gas, such as nitrogen, argon, helium, and the like. For example, when a silicalite is used as catalyst, the inert gas may be used in an amount of about 5 to 95 mol %, preferably about 25 to 35 mol % based on the total feed. Likewise, if the reactants are liquid at the reaction temperature, then they also can be used either alone or with a suitable diluent, e.g. sulfolane or a paraffinic hydrocarbon containing, for example, about 12 to 18 carbon atoms.

Typically, the catalyst is employed in a fixed bed reactor, e.g. in the shape of an elongated pipe or tube where the reactants, typically in the vapor form, are passed over or through the catalyst. Other reactors, such as fluid or ebullient bed reactors, can be employed, if desired. In some instances, it is advantageous to use the catalyst in conjunction with an inert material such as glass wool to regulate the pressure drop of the reactant stream through the catalyst bed and the contact time of the reactant compounds with the catalyst particles.

The following examples are illustrative embodiments of this invention. Percent conversion is calculated by dividing the moles of total product times 100 by the moles of phenol fed. The selectivity is calculated by dividing the percent conversion to the 2- or 4-hydroxyphenyl ketone by the percent conversion to the total hydroxyacetophenones.

Example 1

The catalyst utilized was an H-ZSM-5 zeolite prepared by replacing with hydrogen ions all but 500 ppm based on the weight of the zeolite of the sodium ions in a sodium aluminosilicate ZSM-5 catalyst prepared in accordance with U.S. Patent No, 3,702,886, in which the ratio of silica to alumina was about 12. About 4.78g of this catalyst was mixed with 1.28g of glass wool and charged to an oil heated 14 in. (35.6 cm) tubular reactor having an inside diameter of about 1/4 in. (0.64 cm). The length of the catalyst bed after charging was about 4.5 in. (11.4 cm).

A feed liquid was prepared by mixing 23.5g (0.25 mole) of phenol with 60.0g (1.0 mole) of acetic acid. About 8 ml/hr of the reaction feed liquid was evaporated and charged to the reactor with an average flow of 187 ml/min. of helium carrier gas at a temperature of 245-249° C. and pressures before and after the catalyst bed of 244/240 (before/after) psig (1682/1655 kPa gauge) to 269/257 psig (1855/1772 kPa gauge). The vapor effluent was condensed and collected. After four hours the liquid feed was stopped and the passage of helium carrier gas was continued for another 1 1/2 hours to yield 30.50g of product condensate. Analysis of the condensate indicated the percent conversion to all products including phenyl acetate to be 20.5% and the conversion to hydroxyacetophenones to be 14.5%. Based on the total amount of hydroxyacetophenones produced, the selectivity of 2-HAP was 98.7% and of 4-HAP was 1.3%.

Examples 2 to 5 illustrate the activity of an H-ZSM-5 zeolite catalyst bed over a period of time.

Example 2

The procedure of Example 1 was followed except that about 4.75g of the H-ZSM-5 zeolite mixed with 1.35g of glass wool and charged to the tube reactor, the feed liquid contained 18.8g (0.2 mole) of phenol and 120.0g (2.0 mole) of acetic acid, the average flow of the helium carrier gas was 417 ml./mm., the temperature was in the range of 243-249° C., the pressure before and after the catalyst bed was in the range of 232/222 psig (1560/1531 kPa gauge) to 244/242 psig (1682/1669 kPa gauge), the reactant flow was continued for about 3 hours, and the heating was continued for 2 1/4 hours after, and the helium flow for about sixteen hours after the reactant feed flow was stopped. Condensate product in an amount of 24.53g was collected which on analysis indicated percent conversions to all products including phenyl acetate of 37.7% and to hydroxyacetophenones of 13.2%. Based on the total hydroxyacetophenones, the selectivity of 2-HAP was 100%.

Example 3

The procedure of Example 2 was followed with the same catalyst bed except that the average helium flow was 321.5 ml./min. for the first hour and under 50 ml./min. for the remainder of the flow period due to plugging, the temperature was in the range of 245-250° C., the pressure before and after the catalyst bed was in the range of 104/90 psig (717/621 kPa gauge) to 186/168 psig (1282/1158 kPa gauge) and the heating was continued for 2 3/4 hours after the reactant feed was stopped. The amount of condensate product collected was 24.53g which analyzed to 31.3% conversion to all products including phenyl acetate,

6.5% conversion to hydroxyacetophenones, and selectivities to 2-HAP of 96.3% and to 4-HAP of 3.7%, both based on the total conversion to hydroxyacetophenones.

Example 4

The procedure of Example 2 was followed with the same catalyst bed except that the average helium flow was 198 ml./min., the temperature varied from 295 to 303° C., the pressure before and after the catalyst bed varied from 109/95 psig (752/655 kPa gauge) to 113/95 psig (779/655) kPa gauge), and the heating was continued for 1 1/2 hours and the helium flow for over 60 hours after the reactant feed stream was stopped. Condensate product was collected in an amount of 24.15g which on analysis indicated conversions to all products of 36.6% and to hydroxyacetophenones of 7.0% with selectivities to 2-HAP of 90.9% and 4-HAP of 9.1% based on the total hydroxyacetophenones.

Example 5

The procedure of Example 2 was followed with the same catalyst bed except that the average helium flow was 253.75 ml./min., the temperature was in the range of 295 to 303° C., the pressure before and after the catalyst bed varied from 115/105 psig (792/724 kPa gauge) to 118/105 psig (813/724 kPa gauge), the reactant flow was continued for about 4 hours, and 33.54g of condensate product was collected shortly after the reactant feed stream was stopped. Analysis of the product indicated conversions to all products of 35.0% and to hydroxyacetophenones of 3.1% with selectivities to 2-HAP of 87.0% and 4-HAP of 13.0% based on the total hydroxyacetophenones produced.

Examples 6 to 8

These examples illustrate the effect of contact time of the reactants over catalyst bed on the product selectivity.

The procedure of Example 2 was followed except for the variation of the reactor pressure and helium carrier gas flow, the liquid feed was pumped into the vaporizer at the rate of 4 ml/hr and the reaction was run for 4 hours. Various other parameters and percent conversions to products are tabulated in Table III. It is apparent from these results that shorter contact times afford higher conversions to 2-HAP.

TABLE III - EFFECT OF CONTACT TIME ON THE PRODUCT SELECTIVITY

| Example | Helium Pressure Range (psig) [kPa gauge] before/after | before/after | Average Helium Flow (ml/min) | Contact Time (Secs.) | % Conv. To All Products | % Conv. To HAPS | % Selectivity 2-HAP | 4-HAP | Amt. of Condensat (g) |
|---|---|---|---|---|---|---|---|---|---|
| 6 | 230/212 [1586/1462] | 240/229 [1655/1579] | 109.3 | 58.2 | 31 | 8.7 | 100 | 0 | 11.51 |
| 7 | 205/203 [1413/1400] | 235/231 [1620/1593] | 103 | 42.1 | 40 | 10.4 | 100 | 0 | 14.68 |
| 8 | 75/74 [517/510] | 85/84 [586/579] | 65.4 | 21.7 | 48 | 14.4 | 100 | 0 | 17.65 |

Examples 9 to 13

These Examples illustrate the effect of temperature on the product selectivity using an H-ZSM-5 zeolite as catalyst.

The procedure of Example 2 was followed except that the average helium flow was between 55-63 ml/min., the pressure before and after the catalyst bed varied from 245/244 psig (1689/1682 kPa gauge) to 285/280 psig (1965/1931 kPa gauge), and the liquid feed was pumped in to the vaporizer at the rate of 4 ml/hr. The temperature of the reactor was varied in each of the examples as shown in Table IV which also shows the amounts of condensate product collected and the percent conversion of phenol to all products including phenyl acetate, and to hydroxyacetophenones (HAPS).

TABLE IV

| EFFECT OF TEMPERATURE ON THE ACETYLATION OF PHENOL TO 2-HAP | | | | | | |
|---|---|---|---|---|---|---|
| Example | Temp. °C. | % Conv. To All Prod. | % Conv. To HAPS | % Selectivity | | Amt. of Condensate (g) |
| | | | | 2-HAP | 4-HAP | |
| 9 | 200 | 40.0 | 8.0 | 100 | 0 | 16.35 |
| 10 | 220 | 41.2 | 9.4 | 100 | 0 | 15.81 |
| 11 | 250 | 40.0 | 12.8 | 100 | 0 | 16.87 |
| 12 | 270 | 44.6 | 16.2 | 100 | 0 | 16.61 |
| 13 | 300 | 46.0 | 12.7 | 100 | 0 | 17.40 |

Example 14

The procedure of Example 1 was followed except that about 4.81g of the H-ZSM-5 in conjunction with 1.21g of glass wool was charged to the tube reactor. The feed liquid contained 9.4g (0.1 mole) of phenol and 74.0g (1.0 mole) of propionic acid, and the average flow of the helium carrier gas was 175 ml/min. The temperature was in the range of 246-252° C, and the pressure before and after that catalyst bed was in the range of 90/86 psig (621/593 kPa gauge) to 140/140 psig (965/965 kPa gauge). After four hours the liquid feed was stopped and the passage of helium carrier gas was continued for another 1 1/2 hours to yield 28.9g of product condensate. Analysis of the condensate indicated the percent conversion to all products including phenyl propionate to be 40.0% and the conversion to hydroxypropiophenones to be 3.0%. Based on the total amount of hydroxypropiophenones produced, the selectivity to 2-hydroxypropiophenone was 100%.

Example 15

The procedure of Example 1 was followed except that about 4.76g of the H-ZMS-5 in conjunction with 1.25 g of glass wool was charged to the tube reactor, and the feed liquid contained 9.4g (0.1 mole) of phenol and 88.0g (1.0 mole) of n-butyric acid. The average flow of the helium carrier gas was 112 ml/min., the temperature was in the range of 245-254° C, and the pressure before and after the catalyst bed was in the range of 90/89 psig (620/614 kPa gauge) to 95/94 psig (655/648 kPa gauge). After four hours the liquid feed was stopped and the passage of helium carrier gas was continued for another 1 1/2 hours to yield 33.92g of product condensate. Analysis of the condensate indicated the percent conversion to all products including phenyl n-butyrate to be 30.0% and the conversion to hydroxy n-butyrophenones to be 2.0%. Based on the total amount of hydroxy n-butyrophenones produced, the selectivity to 2-hydroxy n-butyrophenone was 44%, and to 4-hydroxy-n-butyrophenone was 56%.

Examples 16 and 17 illustrate the effect of water on the product selectivity when an H-ZSM-5 zeolite is employed.

Example 16

The procedure of Example 1 was followed except that about 4.86g of the catalyst together with 1.26g of glass wool was charged to the tube reactor. The feed liquid contained 9.4g (0.1 mole) of phenol, 60.0g (1.0 mole) of acetic acid, and 1.8g (0.1 mole) of water. The average flow of the helium carrier gas was 328.4 ml/min. The temperature was in the range of 248-251° C, and the pressure before and after the catalyst bed was in the range of 125/120 psig (862/827 kPa gauge) to 108/102 psig (745/703 kPa gauge). After four hours the liquid feed was stopped and the passage of helium carrier gas was continued for another 1 1/2 hours to yield 36.48g of product condensate. Analysis of the condensate indicated the percent conversion

to all products including phenyl acetate to be 17.0% and the yield to hydroxyacetophenones to be 4.0%. Based on the total amount of hydroxyacetophenones produced,the selectivity to 2-HAP was 100%.

Example 17

The procedure of Example 1 was followed except that about 4.80g of catalyst together with 1.12g of glass wool was charged to the tube reactor. The feed liquid contained 9.4g (0.1 mole) of phenol, 60.0g (1.0 mole) of acetic acid, and 3.6g (0.2 mole) of water. The average flow of the helium carrier gas was 295.7 ml/min. The temperature was in the range of 249-251° C, and the pressure before and after the catalyst bed was in the range of 130/120 psig (896/827 kPa gauge) to 95/94 psig (655/648 kPa gauge). After four hours the liquid feed was stopped and the passage of helium carrier gas was continued for another 1 1/2 hours to yield 39.31g of product condensate. Analysis of the condensate indicated the percent conversion of all products including phenyl acetate to be 21.0% and the yield to hydroxyacetophenones to be 13.0%. Based on the total amount of hydroxyacetophenones produced, the selectivity to 2-HAP was 100%.

The following example illustrates the acetylation of phenol by means of the process of this invention utilizing a slurry phase of H-ZSM-5 zeolite catalyst in an autoclave.

Example 18

A mixture of phenol (9.4g, 0.1 mole) and acetic acid (60.0g, 1.0 mole) in a glass liner was slurried with 9.9g of the catalyst of Example 1 and the liner was inserted into an autoclave. The autoclave was sealed, leak tested, heated to 250°C (in about 30 mins.) with stirring and maintained at this temperature for 1 hour. At the end of this period, the autoclave was cooled, the volatile gases were vented off, and the liquid slurry was removed. Filtration of this mixture gas gave 48.4g of clear liquid. Analysis of this liquid indicated the percent conversion to all products including phenyl acetate to be 31.3% and the yield to hydroxyacetophenones to be 7.0%. Based on the total amount of hydroxyacetophenones produced, the selectivity to 2-HAP was 53.0%, and to 4-HAP was 47.0%.

Examples 19 to 24

These examples illustrate the production of 2-hydroxyacetophenone (2-HAP) from phenol (PhOH) and acetic acid (HOAc) using a silicalite catalyst.

The catalyst used was a silicalite sold by Union Carbide Corporation under the designation "S-115". It was prepared as described in U.S. Patent 4,061,724 and was composed of more than 99 wt. % of silica containing about 6500 to 7000 ppm of alumina such that the $SiO_2$ to $Al_2O_3$ ratio was about 241 and the catalyst contained about 0.03 wt.% of the total of sodium and potassium.

The crystal structure of the silicalite was made up of a tetrahedral framework, which contained a large fraction of five-membered rings of silicon-oxygen tetrahedra. Its channel system was composed of near-circular zig-zag channels (free cross-section 5.4 ± 0.2A) cross-linked by elliptical straight channels with a free cross-section of 5.75 x 5.15 A. Both channels were defined by 10 rings. The X-ray powder diffraction pattern was as defined in Table A of U.S. Patent No. 4,061,724.

Other properties of the silicalite were a pore volume of about 0.19 cc/gm and a crystal density of about 1.76 cc/gm.

The 2-HAP product was produced by the acetylation of phenol with acetic acid in vapor phase over the silicalite catalyst at 300° C and 7 psig (48 kPa gauge) total pressure. The reaction mixture (phenol and acetic acid) was fed by means of a Milton Roy liquid delivery pump at about 0.26 ml/min into a flasher where the mixture was vaporized at 180° C into a stream of nitrogen (50 cc/min). The vapor mixture was carried through stainless steel electrically heated lines to the 0.364 inch (9.25 mm) i.d. U-tube stainless steel reactor immersed in a sandbath. The reactor pressure was maintained constant by means of a back pressure regulator. The catalyst volume was varied from 14 to 56 cc as -20 +30 mesh particles. The reactor effluent was condensed in a chilled water condenser followed by two dry ice-isopropanol traps, weighted and analyzed by a flame ionization GC using a Carbowax 20M capillary column. Water in the liquid product was determined by the Karl Fisher method. The permanent gases were collected in plastic bags and analyzed by mass spectroscopy. The total dry vent was estimated by measuring periodically the flow rate with a soap flowmeter. The duration of a run was in general about 120 minutes. Before every run the microunit was purged with nitrogen to remove any traces of air.

The process conditions varied in the following examples were catalyst volume and age, total vapor feed flow rate, catalyst contact time under the conditions of reaction (CT. TIME) and ratio of phenol to acetic acid

in the reaction mixture. Table V shows the specific conditions of the examples as well as the results obtained including phenol conversion, selectivities of the various product components based on phenol conversion, the space time yield (STY) of 2-HAP and the mass and carbon accountabilities.

Percent conversion is calculated by dividing the moles of phenol reacted times 100 by the moles of phenol fed. The percent selectivity is circulated by dividing the moles of the specific product formed times 100 by the moles of phenol converted. The percent mass accountability (ACCT.% MASS) is the weight of liquid and gas product divided by the weight of liquid fed times 100. The reacted carbon accountability (ACCT.% CARBON) is the total moles of carbon containing products formed divided by the total moles of reactants converted times 100. In addition to 2-HAP and 4-HAP, Table V shows the selectivities to phenyl acetate (PhOAc), 4-acetoxyacetophenone (4-AAP), 2-methylchromone (2-MCH) and 4-methylcoumarin (4-MC). Finally small amounts of by-products (in addition to those mentioned in the tables) such as acetone, carbon dioxide, carbon monoxide and water were also formed.

TABLE V

| EXAMPLE | CATALYST VOL. CC | CATALYST AGE HRS | FLOW RATE (TOTAL) CC/MIN | CT. TIME, SEC | FEED, MOLE % PHOH | FEED, MOLE % HOAC | FEED, MOLE % N2 | PHOH CONV. | SELECTIVITY % 2-HAP | SELECTIVITY % 4-HAP | SELECTIVITY % PHOAC | SELECTIVITY % 2-AAP | SELECTIVITY % 2-MCH | SELECTIVITY % 4-MC | 2-HAP STYL G/L/HR | ACCT. % MASS | ACCT. % CARBON |
|---|---|---|---|---|---|---|---|---|---|---|---|---|---|---|---|---|---|
| 19 | 56 | 9.7 | 137.9 | 18.7 | 31.1 | 31.2 | 37.0 | 31.1 | 59.2 | 3.5 | 38.3 | 0.3 | 0.1 | 0.1 | 47.0 | 100.7 | 100.5 |
| 20 | 14 | 1.3 | 136.7 | 4.7 | 31.5 | 31.6 | 36.3 | 28.1 | 45.2 | 3.5 | 37.0 | 0.4 | 0.1 | 0.1 | 130.4 | 99.4 | 90.8 |
| 21 | 28 | 4.3 | 145.8 | 8.8 | 32.5 | 32.5 | 34.6 | 29.6 | 48.9 | 2.8 | 35.0 | 0.4 | 0.1 | 0.1 | 81.4 | 90.9 | 93.8 |
| 22 | 28 | 3.0 | 153.9 | 8.4 | 5.9 | 61.0 | 32.0 | 66.6 | 47.4 | 2.9 | 32.3 | 1.0 | 0.4 | 0.1 | 32.1 | 99.9 | 93.2 |
| 23 | 28 | 3.8 | 150.4 | 8.6 | 11.1 | 55.3 | 33.0 | 54.1 | 45.7 | 2.9 | 33.7 | 0.8 | 0.2 | 0.1 | 50.2 | 97.2 | 81.6 |
| 24 | 28 | 1.1 | 145.3 | 8.9 | 21.4 | 42.9 | 34.6 | 40.0 | 45.1 | 2.4 | 29.0 | 0.3 | 0.1 | 0.1 | 66.5 | 94.5 | 86.4 |

Examples 25 to 31

The procedure of Examples 19 to 24 was followed except that the feed reactant was phenyl acetate either in the presence of acetic acid additive or solvent (Examples 25 to 29) or without any acetic acid (Examples 30 and 31), and catalyst volume was 28cc. the process conditions and results of these examples

are shown in Table VI, wherein the percent conversions and selectivities are based on phenyl acetate.

TABLE VI

| EXAMPLE | CATALYST AGE/HRS | FEED, MOLE % HOAC | FEED, MOLE % PHOAC | FEED, MOLE % H2O | FEED, MOLE % N2 | CT. TIME SEC | CONV. % | SELECTIVITY % 2-HAP | SELECTIVITY % 4-HAP | SELECTIVITY % 4-AAP | SELECTIVITY % 2-MCh | SELECTIVITY % PHOH | 2-HAP STY G/L/HR | ACC % MASS | ACC % C |
|---|---|---|---|---|---|---|---|---|---|---|---|---|---|---|---|
| 25 | 1.3 | 56.8 | 5.5 | 0.5 | 37.2 | 8.2 | 67.8 | 43.6 | 3.9 | 3.1 | 0.4 | 37.0 | 30.5 | 93.5 | 99.2 |
| 26 | 4.2 | 59.0 | 5.7 | 0.5 | 34.7 | 8.5 | 54.7 | 30.6 | 6.6 | 10.4 | 2.1 | 29.8 | 17.8 | 96.7 | 77.7 |
| 27 | 6.2 | 28.0 | 27.8 | 0.3 | 44.0 | 10.5 | 20.7 | 12.4 | 6.1 | 27.9 | 0.3 | 9.8 | 10.4 | 96.8 | 64.0 |
| 28 | 1.1 | 27.8 | 27.6 | 0.3 | 44.3 | 10.5 | 44.1 | 25.5 | 9.9 | 17.6 | 0.3 | 35.4 | 45.3 | 94.2 | 85.8 |
| 29 | 3.1 | 27.9 | 27.7 | 0.3 | 44.0 | 10.4 | 31.3 | 17.8 | 9.4 | 21.4 | 0.2 | 40.5 | 22.8 | 101.7 | 88.2 |
| 30 | 1.4 | 0 | 49.6 | 0.4 | 50.0 | 12.9 | 22.9 | 14.4 | 11.1 | 24.1 | 0.2 | 24.0 | 19.4 | 101.3 | 76.5 |
| 31 | 3.8 | 0 | 49.8 | 0.4 | 49.8 | 12.9 | 9.1 | 9.1 | 5.7 | 26.5 | 0.4 | 17.8 | 4.9 | 99.3 | 64.3 |

Examples 32 and 33 illustrate the effect of a silica binder on the catalytic activity of a S-115 catalyst in the form of 1/8 inch (3.2 mm) extrudates containing such a binder.

Example 32

The procedure of Examples 25 to 31 was followed except that the catalyst was the S-115 used in those examples blended with 20 wt. % conventional silica (as binder) end containing 0.2 wt. % of the total of sodium and potassium, said weight percents being based on the total catalyst weight. The catalyst volume was 28cc and its age was 1.1 hours. The feed flowed at a rate of 118.9 cc/min and was composed of 28.9 mol % of phenyl acetate, 29.1 mol % of acetic acid and 42.0 mol % nitrogen and the catalyst contact time was 10.8 sec. The reaction resulted in a percent conversion of phenyl acetate of 17.8% at the following selectivities: 2-HAP 2.3%; 4-HAP 9.0%; 4-AAP 16.0%; and PhoH 78.7%; an STY to 2-HAP of 1.7 g/liter/hr, a mass accountability of 99.6%; and a reacted carbon accountability of 91.9%.

Example 33

The procedure of Examples 19 to 24 and 32 was followed except that the catalyst was treated with HCl such that the total of the sodium and potassium content was 0.1 wt.%, the alumina was 5500 ppm and the $SiO_2$ to $Al_2O_3$ ratio was about 307. The catalyst age at the beginning of the reaction was 1.0 hours, the feed flowed at a rate of 137.4 cc/min and was composed of 31.4 mol % of phenol, 32.1 mol % of acetic acid and 36.5 mol % of nitrogen, and the catalyst contact time was 9.4 sec. The reaction resulted in a percent conversion of phenol of 27.5 % at the following percent selectivities: 2-HAP 3.4%; 4-HAP 5.2%; 4-AAP 1.4%; 2-MCH 0.1%; and PhOAc 75.8%; an STY to 2-HAP of 4.8 g/liter/hr, a mass accountability of 100.3% and a reacted carbon acccuntability of 93.8%.

## Claims

**1.** A process for the production of a 2-hydroxyphenyl lower alkyl ketone by reaction of phenol with a lower alkanoic acid comprising contacting a feed stream comprising said phenol and acid at an elevated temperature with an H-ZSM-5 zeolite or silicalite catalyst.

**2.** The process of claim 1 wherein the catalyst is an H-ZSM-5 zeolite having the formula:

$$0.9 \pm 0.2\ M_{2/n}0:W_2O_3:5\text{-}100\ YO_2:zH_2O$$

wherein M is a cation, n is the valence of said cation, W is selected from the group consisting of aluminum and gallium, Y is selected from the group consisting of silicon and germanium, and Z is from 0 to 40, and in which at least about 80% of the cations are replaced with hydrogen ions.

**3.** The process of claim 2 wherein said ZSM-5 zeolite has an X-ray diffraction pattern with lines as shown in Table I of the specification.

**4.** The process of claim 2 or 3 where said catalyst has the formula:

$$0.9 \pm 0.2\ M_{2/n}O:Al_2O_3:5\text{-}100\ SiO_2:zH_2O$$

and M is selected from the group consisting of alkali metal cations and tetraalkylammonium cations, the alkyl groups of which contain 2-5 carbon atoms.

**5.** The process of claim 4 wherein the ratio of $SiO_2$ to $Al_2O_3$ in said catalyst is in the range of about 10 to 60.

**6.** The process of any of claims 1 to 5, wherein said reaction occurs in the vapor phase and said elevated temperature is in the range of 160 to 350 °C.

**7.** The process of claim 6 wherein said temperature is in the range of 200 to 300 °C.

**8.** The process of any of claims 1 to 7 wherein said feed stream also contains 0.5 to 2 moles of water per mole of phenol.

**9.** The process of claim 1 wherein the catalyst is a silicalite catalyst containing 700 to 14000 ppm of

alumina, said catalyst having been calcined from the as synthesized form at least once, to produce a product comprising a 2-hydroxyphenyl lower alkyl ketone.

**10.** The process of claim 9 wherein said calcined silicalite contains 5000 to 7000 ppm of alumina.

**11.** The process of claim 9 or 10 wherein said calcined silicalite has a mean refractive index of 1.39 ± 0.01 and a specific gravity at 25 ° C of 1.70 ± 0.08.

**12.** The process of claim 9, 10 or 11 wherein the six strongest d-values of the X-ray powder diffraction pattern of said calcined silicalite are as set forth in the following table wherein "S" = strong and "VS" = very strong:

| d-A | Relative intensity |
|---|---|
| 11.1 ± 0.2 | VS |
| 10.1 ± 0.2 | VS |
| 3.85 ± 0.07 | VS |
| 3.82 ± 0.07 | S |
| 3.76 ± 0.05 | S |
| 3.72 ± 0.05 | S |

**13.** The process of any of claims 9 to 12, wherein the reaction occurs in the vapour phase and said elevated temperature is in the range of 200 to 350 ° C.

**14.** The process of any of claims 1 to 13, wherein the ratio of phenol to alkanoic acid in the feed stream is from 100 : 1 to 1 : 100.

**15.** The process of any of claims 1 to 14 wherein said catalyst is in the form of a fixed bed and said feed stream into said bed also contains an inert carrier gas.

**16.** The process of claim 15 wherein said inert gas is nitrogen.

**17.** The process of any of claims 1 to 16 wherein said lower alkanoic acid is acetic acid and said 2-hydroxyphenyl ketone is 2-hydroxyacetophenone.

**18.** The process of any of claims 1 and 9 to 13, modified in that said 2-hydroxyphenyl lower alkyl ketone is produced by contacting an ester of phenol and a lower alkanoic acid with a silicalite catalyst at an elevated temperature, wherein said feed stream comprises said ester and may optionally contain free lower alkanoic acid, whereby said 2-hydroxyphenyl lower alkyl ketone is produced by the Fries rearrangement of said ester.

**19.** The process of claim 18 wherein phenyl acetate is reacted in the presence of free acetic acid.

## Revendications

**1.** Procédé pour la fabrication d'une cétone 2-hydroxyphényl alkyle inférieure par réaction d'un phénol avec un acide alcanoïque inférieur, comprenant la mise en contact d'un courant d'alimentation comprenant ledit phénol et ledit acide à une température élevée avec un catalyseur de silicalite ou de zéolite H-ZSM-5.

**2.** Procédé de la revendication 1 où le catalyseur est une zéolite H-ZSM-5 ayant pour formule :

$$0{,}9 \pm 0{,}2\ M_{2/n}O:W_2O_3:5\text{-}100\ YO_2:zH_2O$$

où M est un cation, n est la valence dudit cation, W est choisi dans le groupe consistant en aluminium et gallium, Y est choisi dans le groupe consistant en silicium et germanium et z est compris entre 0 et 40 et où au moins 80% des cations sont remplacés par des ions d'hydrogène.

3. Procédé de la revendication 2 où ladite zéolite ZSM-5 a un schéma de diffraction des rayons X avec des lignes telles que montrées au Tableau 1 de la description.

4. Procédé selon la revendication 2 ou 3 où ledit catalyseur a pour formule :

$0,9 \pm 0,2\ M_{2/n}O:Al_2O_3:5\text{-}100\ SiO_2:zH_2O$

et M est choisi dans le groupe consistant en cations d'un métal alcalin et cations de tétraalkylammonium, dont les groupes alkyles contiennent 2-5 atomes de carbone.

5. Procédé de la revendication 4 où le rapport de $SiO_2$ à $Al_2O_3$ dans ledit catalyseur est compris entre environ 10 et 60.

6. Procédé selon l'une quelconque des revendications 1 à 5,où ladite réaction se produit en phase vapeur et ladite température élevée est comprise entre 160 et 350 ° C.

7. Procédé de la revendication 6 où ladite température est comprise entre 200 et 300 ° C.

8. Procédé selon l'une quelconque des revendications 1 à 7 où ledit courant d'alimentation contient également 0,5 à 2 moles d'eau par mole de phénol.

9. Procédé de la revendication 1 où le catalyseur est un catalyseur de silicalite contenant 700 à 14.000 ppm d'alumine, ledit catalyseur ayant été calciné à partir de la forme telle que synthétisée au moins une fois, pour donner un produit comprenant une cétone 2-hydroxyphényl alkyle inférieure.

10. Procédé de la revendication 9 où ladite silicalite calcinée contient 5.000 à 7.000 ppm d'alumine.

11. Procédé de la revendication 9 ou 10 où ladite silicalite calcinée a un indice moyen de réfraction de 1,39 ± 0,01 et une densité à 25 ° C de 1,70 ± 0,08.

12. Procédé de la revendication 9, 10 ou 11 où les six valeurs les plus fortes du schéma de diffraction aux rayons X des poudres de ladite silicalite calcinée sont telles qu'indiquées au tableau qui suit où "S" = forte et "VS" = très forte :

| d-A | Intensité relative |
|---|---|
| 11,1 ± 0,2 | VS |
| 10,1 ± 0,2 | VS |
| 3,85 ± 0,07 | VS |
| 3,82 ± 0,07 | S |
| 3,76 ± 0,05 | S |
| 3,72 ± 0,05 | S |

13. Procédé selon l'une quelconque des revendications 9 à 12, où la réaction se produit dans la phase vapeur et ladite température élevée est comprise entre 200 et 350 ° C.

14. Procédé selon l'une quelconque des revendications 1 à 13, où le rapport du phénol à l'acide alcanoïque dans le courant d'alimentation est compris entre 100:1 et 1:100.

15. Procédé selon l'une quelconque des revendications 1 à 14 où ledit catalyseur a la forme d'un lit fixe et ledit courant d'alimentation dans ledit lit contient également un gaz porteur inerte.

16. Procédé de la revendication 15 où ledit gaz inerte est de l'azote.

17. Procédé selon l'une quelconque des revendications 1 à 16 où ledit acide alcanoïque inférieur est l'acide acétique et ladite 2-hydroxyphényl cétone est la 2-hydroxyacétophénone.

18. Procédé selon l'une quelconque des revendications 1 et 9 à 13, modifié par le fait que ladite cétone 2-hydroxyphényl alkyle inférieure est produite par mise en contact d'un ester de phénol et d'un acide alcanoïque inférieur avec un catalyseur de silicalite à une température élevée où ledit courant d'alimentation comprend ledit ester et peut facultativement contenir de l'acide alcanoïque inférieur libre, ainsi ladite cétone 2-hydroxyphényl alkyle inférieure est produite par le réarrangement de Fries dudit ester.

19. Procédé de la revendication 18 où l'acétate de phényle réagit en présence de l'acide acétique libre.

## Patentansprüche

1. Verfahren zur Herstellung eines 2-Hydroxyphenyl-niedrig-Alkylketons durch Umsetzen von Phenol mit einer niedrig-Alkansäure, dadurch gekennzeichnet, daß ein Beschickungsstrom, bestehend aus dem Phenol und Säure bei erhöhter Temperatur mit einem H-ZSM-5 Zeolith- oder Silicalitkatalysator in Berührung gebracht wird.

2. Verfahren nach Anspruch 1, dadurch gekennzeichnet, daß der Katalysator ein H-ZSM-5 Zeolith mit der Formel:

$$0,9 \pm 0.2\ M_{2/n}0:W_2O_3:5\text{-}100\ YO_2:zH_2O$$

ist, worin M ein Kation ist, n die Wertigkeit dieses Kations ist, W aus der Gruppe, bestehend aus Aluminium und Gallium, ausgewählt ist, Y aus der Gruppe, bestehend aus Silizium und Germanium, ausgewählt ist und z von 0 bis 40 ist und in welchem zumindest 80% der Kationen durch Wasserstoffionen ersetzt sind.

3. Verfahren nach Anspruch 2, dadurch gekennzeichnet, daß der ZSM-5 Zeolith ein Röntgenbeugungsmuster mit wie in Tabelle I der Beschreibung gezeigten Linien hat.

4. Verfahren nach Anspruch 2 oder 3, dadurch gekennzeichnet, daß der Katalysator die Formel

$$0.9 \pm 0.2\ M_{2/n}O:Al_2O_3:5\text{-}100\ SiO_2:zH_2O$$

hat und M aus der Gruppe, bestehend aus Alkalimetallkationen und Tetraalkylammoniumkationen, deren Alkylgruppen 2-5 Kohlenstoffatome enthalten, ausgewählt ist.

5. Verfahren nach Anspruch 4, dadurch gekennzeichnet, daß das Verhältnis von $SiO_2$ zu $Al_2O_3$ im Katalysator im Bereich von etwa 10 bis 60 liegt.

6. Verfahren nach einem der Ansprüche 1 bis 5, dadurch gekennzeichnet, daß die Reaktion in der Dampfphase stattfindet und die erhöhte Temperatur im Bereich von 160 bis 350 °C liegt.

7. Verfahren nach Anspruch 6, dadurch gekennzeichnet, daß die Temperatur im Bereich von 200 bis 300 °C liegt.

8. Verfahren nach einem der Ansprüch 1 bis 7, dadurch gekennzeichnet, daß der Beschickungsstrom auch 0,5 bis 2 Mol Wasser pro Mol Phenol enthält.

9. Verfahren nach Anspruch 1, dadurch gekennzeichnet, daß der Katalysator ein Silicalitkatalysator, enthaltend 700 bis 14000 ppm Tonerde, ist, wobei der Katalysator aus der synthetisierten Form zumindest ein Mal kalziniert wurde, um ein Produkt herzustellen, welches aus einem 2-Hydroxyphenyl-niedrig-Alkylketon besteht.

10. Verfahren nach Anspruch 9, dadurch gekennzeichnet, daß der kalzinierte Silicalit 5000 bis 7000 ppm Tonerde enthält.

11. Verfahren nach Anspruch 9 oder 10, dadurch gekennzeichnet, daß der kalzinierte Silicalit einen mittleren Brechungsindex von 1,39 ± 0,01 und bei 25 °C ein spezifisches Gewicht von 1,70 ± 0,08

besitzt.

12. Verfahren nach Anspruch 9, 10 oder 11, dadurch gekennzeichnet, daß die sechs stärksten d-Werte des Röntgen-Pulverbeugungsmusters des kalzinierten Silicalits wie in der folgenden Tabelle angeführt, worin "S" = stark und "SS" = sehr stark ist, sind:

| d-A | relative Intensität |
|---|---|
| 11,1 ± 0,2 | SS |
| 10,1 ± 0,2 | SS |
| 3,85 ± 0,07 | SS |
| 3,82 ± 0,07 | S |
| 3,76 ± 0,05 | S |
| 3,72 ± 0,05 | S |

13. Verfahren nach einem der Ansprüche 9 bis 12, dadurch gekennzeichnet, daß die Reaktion in der Dampfphase stattfindet und die erhöhte Temperatur im Bereich von 200 bis 350 °C liegt.

14. Verfahren nach einem der Ansprüche 1 bis 13 , dadurch gekennzeichnet, daß das Verhältnis von Phenol zur Alkansäure im Beschickungsstrom 100:1 bis 1:100 beträgt.

15. Verfahren nach einem der Ansprüche 1 bis 14, dadurch gekennzeichnet, daß der Katalysator in Form eines Festbettes ist und der Beschickungsstrom in das Bett auch ein inertes Trägergas enthält.

16. Verfahren nach Anspruch 15, dadurch gekennzeichnet, daß das inerte Gas Stickstoff ist.

17. Verfahren nach einem der Ansprüche 1 bis 16, dadurch gekennzeichnet, daß die niedrig-Alkansäure Essigsäure ist und das 2-Hydroxyphenylketon 2-Hydroxyacetophenon ist.

18. Verfahren nach einem der Ansprüche 1 und 9 bis 13, insofern abgeändert, als das 2-Hydroxyphenyl-niedrig-Alkylketon hergestellt wird, indem ein Ester von Phenol und einer niedrig-Alkansäure mit einem Silicalitkatalysator bei erhöhter Temperatur in Berührung gebracht wird, wobei der Beschickungsstrom aus dem Ester besteht und gegebenenfalls freie niedrig-Alkansäure enthalten kann, wobei das 2-Hydroxyphenyl-niedrig-Alkylketon durch die Fries Umlagerung des Esters hergestellt wird.

19. Verfahren nach Anspruch 18, dadurch gekennzeichnet, daß Phenylacetat in Gegenwart freier Essigsäure umgesetzt wird.